# EUROPEAN PATENT APPLICATION

(11) **EP 4 477 258 A1**
(43) Date of publication of application: **18.12.2024**
(21) Application number: 23752942.5
(22) Date of filing: 09.02.2023
(51) Int. Cl.: A61N 5/06

(54) **METHOD FOR IMPROVING PHYSIOLOGICAL CONDITIONS BY PHOTOSTIMULATION AND DEVICE TO BE USED THEREIN**

(30) Priority: 10.02.2022 JP 2022019580
(71) Applicant: Tsubota Laboratory, Inc., Tokyo 160-0016 (JP)
(72) Inventor: HAYANO Motoshi, Tokyo 160-0016 (JP); TSUBOTA Kazuo, Tokyo 160-0016 (JP)
(74) Representative: Plasseraud IP
(86) International application number: PCT/JP2023/004385
(87) International publication number: WO 2023/153478

(57) **Abstract**

The present disclosure is related to a method for improving physiological state by light stimulation in which light of a specific wavelength such as violet light is irradiated at a specific blinking frequency, and an apparatus used for the same. More specifically, improvement of physiological state such as, improvement or suppression of dermatitis, improvement or suppression of alopecia, improvement of hair condition, improvement or suppression of graying of hair, improvement or suppression of hearing loss, improvement or suppression of vision loss, improvement or enhancement of muscle strength, improvement or suppression of kyphosis, improvement or suppression of pain and/or feeling of discomfort, improvement of autonomic nerve abnormalities, improvement of healthy life expectancy, and obesity control is carried out by irradiating light of a specific wavelength such as violet light to a living body.

## Description

### Technical Field

The present invention relates to a method for improving physiological state by light stimulation and an apparatus used for the same, and more particularly relates to a method for improving physiological state by light stimulation in which light of a specific wavelength such as violet light is irradiated at a specific blinking frequency, and an apparatus used for the same.

### Background Art

Effects of light on a human body have been studied from various perspectives in recent years, and reported on the basis of new findings. For instance, a fact that circadian rhythm is improved by bathing in the sunlight (Non-Patent Document 1), facts such as, light irradiated from a display such as a liquid-crystal display in which LED (light emitting diode) lighting and an LED is used as a backlight, have a substantial effect on body and mind (Non-Patent Document 2), and violet light prevents myopia and suppresses development of myopia (Patent Document 1) have been reported. Recently, in particular, inventors of the present invention have submitted an interesting report about effects of violet light on eyes, and in Patent Document 1 and Non-Patent Document 3 for example, it has been proposed that light of a specific wavelength is effective in preventing myopia and suppressing myopia, and with myopic population still on the rise globally in recent years, high expectations are placed.

Moreover, research and development of various therapeutic techniques has also been active, and research and development of therapeutic techniques with reduced burden on body, particularly by not using medication or reducing the use thereof, has been carried out.

In WO2020/027305A1 (Patent Document 2), an apparatus which carries out control of brain waves and control of cell activity by irradiating light of a specific wavelength constantly or at a specific blinking frequency to a subject has been disclosed. This apparatus includes a light source which irradiates light of a specific wavelength constantly or at a specific blinking frequency and a controller which controls emission of light which induces specific brain waves in the subject having received the light where the brain waves are the same or substantially same as or different from the irradiation state of the light, and it functions as an apparatus controlling brain waves and cell activity by light stimulation.

### Citation List

### Non-patent Documents

Non-Patent Document 1: Anti-aging Medicine by Megumi Hatori and Kazuo Tsubota, Japanese Society of Anti-aging Medicine Magazine Vol. 11, No. 3, 065 (385) -072 (392), (2015)
Non-Patent Document 2: Blue Light Hazard by Kazuo Tsubota, Published by Shueisha on November 20, 2013
Non-Patent Document 3: Hidemasa Torii et al., EBioMedicine, `DOI: http://dx.doi.org/10.1016/j.ebiom.2016.12.007.

### Patent Documents

Patent Document 1: WO2015/186723A1
Patent Document 2: WO2020/027305A1

### Summary of the Invention

### Problems to be Solved by the Invention

The inventors of the present invention discovered that irradiation of light of a specific wavelength, particularly violet light (visible light rays in a region of 360 nm to 400 nm) at a blinking frequency accelerates improvement of a physiological state. The present invention is based on such discovery, and an object thereof is to provide a method for improving physiological state by irradiating light of a specific wavelength such as violet light constantly or at a specific blinking frequency, and an apparatus and a computer program used for the same.

### Means for Solving the Problems

According to one aspect, the present disclosure is related to a method for improving physiological state of a subject which includes irradiating light of a specific wavelength to the subject.

Moreover, according to one aspect, the present disclosure is related to a method for improving physiological state of a subject which includes improving physiological state of the subject by irradiating light of a specific wavelength to the subject by controlling a light irradiating apparatus. In some embodiments, the light irradiating apparatus may include a light source which emits light of a specific wavelength constantly or at a specific blinking frequency to a living body, and a controller which controls the blinking frequency of the light source.

In some embodiments, the improvement of physiological state may be at least one selected from a group consisting of improvement or suppression of dermatitis, improvement or suppression of alopecia, improvement of hair condition, improvement or suppression of graying of hair, improvement or suppression of hearing loss, improvement or suppression of vision loss, improvement or enhancement of muscle strength, improvement or suppression of kyphosis, improvement or suppression of pain and/or feeling of discomfort, improvement of autonomic nerve abnormalities, improvement of healthy life expectancy, and obesity control.

In some embodiments, the light used for irradiation is violet light, and the specific wavelength includes a wavelength in a range of 350 nm to 400 nm, and particularly a wavelength of approximately 380 nm. In some embodiments, the blinking frequency is either 0 Hz or a frequency in a range of 30 Hz to 75 Hz, and a frequency in a range of 35 Hz to 60 Hz, and particularly a frequency of approximately 40 Hz. In some embodiments, irradiation conditions include an irradiation time of the light source, and the irradiation time may be time in an arbitrary range of 10 seconds to 24 hours per day, and may be any one of 10 seconds, 30 seconds, 45 seconds, one minute, three minutes, five minutes, 10 minutes, 15 minutes, 30 minutes, 45 minutes, one hour, two hours, three hours, four ours, five hours, six hours, seven hours, eight hours, nine hours, 10 hours, 11 hours, 12 hours, 18 hours, and 24 hours, or may be an arbitrary time included in a range (for example, a range of one hour to 12 hours) stipulated by the abovementioned arbitrary time. The specific period for which the light is irradiated continuously may be, for example, one day, two days, three days, four days, five days, six days, one week, two weeks, three weeks, one month, two months, three months, six months, one year, two years, three years, or a period longer than these periods.

In some embodiments, the light source may be spectacles with a light source or a spectacle frame with a light source, a desktop light source, a mobile terminal mounted light source, a light source installed nearby or in front of face, a portable light source, an indoor lighting, or a desk lamp, but is not restricted thereto.

In some embodiments, the subject to which light is irradiated is an animal, and particularly, a mammal, and more specifically, a human. Therefore, one embodiment according to the present disclosure is related to a method for improving physiological state which includes irradiating violet light to a human. A site to which the light is irradiated may be, for example, skin, scalp, and eyes.

According to one aspect, the present disclosure is related to an apparatus for improving physiological state by light stimulation having at least one light source which emits light and a driving circuit which drives the light source, and the light emitted by the light source is light of a specific wavelength which produces an effect of improving a physiological state by being irradiated to a living body. In other words, the present disclosure is related to an apparatus for improving physiological state by light stimulation having at least one light source which emits light and a driving circuit which drives the light source, and the driving circuit is configured to control the light source to irradiate light of a specific wavelength which produces an effect of improving physiological state of a subject.

In some embodiments, the driving circuit includes at least one processor which is communicably connected to the light source and at least one memory, and at least one memory for storing commands that are executable by the processor.

According to one aspect, the present disclosure is related to a method of operating an apparatus for improving physiological state by light stimulation having at least one light source which emits light and a driving circuit which drives the light source, and light emitted by the light source is light of a specific wavelength which produces an effect of improving physiological state by being irradiated to a living body, and the apparatus includes a light source which irradiates light of a specific wavelength constantly or at a specific blinking frequency to a living body and a controller which controls the blinking frequency of the light source, which includes controlling the blinking frequency of the light source either to 0 Hz or to a frequency in a range of 30 Hz to 75 Hz by the controller, and irradiating light of a wavelength in a range of 350 nm to 400 nm to the living body by the light source. Moreover, according to one aspect, the present disclosure is related to a method of operating an apparatus for improving physiological state by light stimulation having at least one light source which emits light and a driving circuit which drives the light source, and the driving circuit is configured to control the light source to irradiate light of a specific wavelength which produces an effect of improving physiological state of a subject, and the apparatus includes a light source which irradiates light of a specific wavelength constantly or at a specific blinking frequency to a living body and a controller which controls the blinking frequency of the light source, which includes controlling the blinking frequency of the light source to 0 Hz or to a frequency in a range of 30 Hz to 75 Hz by the controller, and irradiating light of a wavelength in a range of 350 nm to 400 nm to the living body by the light source.

According to one aspect, the present disclosure is related to a computer program which causes an apparatus including a light source which irradiates light of a specific wavelength constantly or at a specific blinking frequency to a living body and a controller which controls the blinking frequency of the light source to execute a method of operating an apparatus for improving physiological state by light stimulation having at least one light source which emits light and a driving circuit which drives the light source, and the light emitted by the light source is light of a specific wavelength which produces an effect of improving physiological state by being irradiated to a living body, and the apparatus includes a light source which irradiates light of a specific wavelength constantly or at a specific blinking frequency to a living body and a controller which controls the blinking frequency of the light source, which includes controlling the blinking frequency of the light source either to 0 Hz or to a frequency in a range of 30 Hz to 75 Hz by the controller, and irradiating light of a wavelength in a range of 350 nm to 400 nm to the living body by the light source. Moreover, according to one aspect, the present disclosure is related to a computer program which causes an apparatus including a light source which irradiates light of a specific wavelength constantly or at a specific blinking frequency to a living body and a controller which controls the blinking frequency of the light source to execute a method of operating an apparatus for improving physiological state by light stimulation having at least one light source which emits light and a driving circuit which drives the light source, and the driving circuit is configured to control the light source to irradiate light of a specific wavelength which produces an effect of improving physiological state of a subject, and the apparatus includes a light source which irradiates light of a specific wavelength constantly or at a specific blinking frequency to a living body and a controller which controls the blinking frequency of the light source, which includes controlling the blinking frequency of the light source to 0 Hz or to a frequency in a range of 30 Hz to 75 Hz by the controller, and irradiating light of a wavelength in a range of 350 nm to 400 nm to the living body by the light source.

In the method and apparatus according to the present disclosure, the light, for example, is violet light. According to this invention, since it is possible to irradiate violet light of a wavelength outside the visible light region to a living body, it is possible to have an effect on the living body without feeling flickering or dazzling as in a case of white light. Moreover, the violet light is light of a wavelength in a range of 360 nm to 400 nm, and light of this wavelength has a low visible sensitivity as compared to that of white light, and is light of a wavelength region which imparts no uncomfortable feeling or hardly any uncomfortable feeling to a living body (particularly human).

In some embodiments of the present disclosure, light of a wavelength in a range of 350 nm to 400 nm, such as light of one of wavelengths 350 nm, 360 nm, 370 nm, 380 nm, 390 nm, or 400 nm, or light of an arbitrary wavelength included in a range stipulated by the abovementioned arbitrary wavelength (for example, in a range of 370 nm to 390 nm) may be used. In some embodiments of the present disclosure, a wavelength of approximately 380 nm is included. Moreover, the term 'approximately' used in the present specification indicates that it includes any value that is within a variation of up to ±5% of the value modified by this term.

In the method and apparatus according to the present disclosure, the condition for irradiation of light is that the light is lit constantly (in other words, 0 Hz) or irradiated at a blinking frequency in a range of higher than 0 Hz up to 150 Hz.

In some embodiments of the present disclosure, for the light which is irradiated constantly (0 Hz) or the light which is irradiated at a blinking frequency in the range of 30 Hz to 75 Hz, for instance, light blinking at any of the blinking frequencies 30 Hz, 35 Hz, 40 Hz, 45 Hz, 50 Hz, 55 Hz, 60 Hz, 65 Hz, 70 Hz, or 75 Hz, or light of an arbitrary blinking frequency included in a range stipulated by any of the abovementioned arbitrary blinking frequencies (for example, in a range of 35 Hz to 45 Hz), may be used. In some embodiments of the present disclosure, the blinking frequency is approximately 40 Hz.

In the method and apparatus according to the present disclosure, the light can be irradiated in a range of irradiance of 0.5 µW/cm² to 1000 µW/cm² for example, or in a range of irradiance of 0.1 µW/cm² (0.001 W/m²) to 5000 µW/cm² (50 W/m²). According to some embodiments, by irradiating light such as violet light in the abovementioned range of irradiance, it is possible to have an effect on physiological state of the subject. Even when it is a weak light of an extremely small amount (light with a weak optical sensitivity) in particular, it has been verified that a characteristic phenomenon may occur.

In the method and apparatus according to the present disclosure, the controller of the apparatus, by transceiving to and from an isolated controller such as a portable terminal, is capable of carrying out control by changing irradiation conditions of light (including irradiating constantly or at a blinking frequency) such as irradiance, irradiation time, irradiation-start time, irradiation-end time, and irradiating constantly or at a blinking frequency. According to some embodiments, the abovementioned irradiation conditions being subjected to isolated control, it is possible to set arbitrarily irradiation conditions appropriate for inducing improvement of physiological state, and have the desired effect.

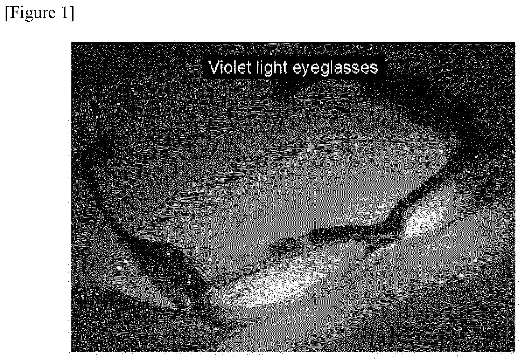

In the method and apparatus according to the present disclosure, the light source may be a light source installed nearby or in front of face, such as spectacles with a light source (for example, refer to Fig. 1) or a spectacle frame with a light source, a desktop light source, and a mobile terminal mounted light source. According to some embodiments, as it is possible to irradiate specific light from the light source installed nearby or in front of face such as spectacles with a light source or spectacle frame with a light source that can be worn easily and have no uncomfortable feeling in daily use, they are highly practical, and are capable of irradiating light any time even in various situations and varied environments.

In the method and apparatus according to the present disclosure, the light source may be a non-mounting type light source such as a portable light source, or may be a mounting type light source such as an indoor lighting, a table stand, and a dedicated device. According to some embodiments, it is possible to make it an apparatus in various forms of light source appropriate for an environment of usage. For instance, the light source may be used in combination with a glass, a spectacle lens, or a contact lens that allows violet light to pass through. Moreover, sunlight that has passed through a glass, a spectacle lens, or a contact lens that allows violet light to pass through, may be used as the light source.

The apparatus according to the present disclosure is an apparatus for improving physiological state by irradiating violet light constantly to a living body, which is characterized by comprising a light source which emits the violet light, and a light-emission time controller which irradiates the violet light for a specific time or a specific period of time.

Furthermore, as it is described below while referring to the accompanying diagrams, in some embodiments of the present disclosure, the method for improving physiological state of a subject, an apparatus for improving physiological state by light stimulation which is configured to irradiate light of a specific wavelength producing an effect of improving physiological state to a living body, a method of operating the apparatus for improving physiological state by light stimulation which is configured to irradiate light of a specific wavelength producing an effect of improving physiological state to a living body, and a computer program which causes to execute the method of operating, are provided.

### Brief Description of the Drawings

Fig. 1 is an example of violet light spectacles which irradiate violet light;
Fig. 2 is a graph showing a relationship of wavelength and spectral irradiance of violet fluorescent light;
Fig. 3 is a light spectrum of an LED with a peak wavelength of 375 nm;
Fig. 4 is a block diagram of an embodiment of an apparatus for controlling biological function according to the present invention;
Fig. 5 is a graph showing results of frailty index test (coat condition, alopecia, dermatitis) at the age of 92 weeks;
Fig. 6 is a graph showing results of frailty index test (forelimb grip strength, kyphosis, total score) at the age of 92 weeks;
Fig. 7 is a graph showing results of frailty index test (alopecia, dermatitis, loss of fur color, coat condition) at the age of 100 weeks;
Fig. 8 is a graph showing results of frailty index test (hearing loss, menace reflex, mouse grimace scale, piloerection) at the age of 100 weeks;
Fig. 9 is a photograph showing a comparison of alopecia and gray hair for mice in a control group (WL) and a VL irradiation group (VL); and
Fig. 10 is a graph showing results of frailty index test (body weight) at the age of 100 weeks.

### Mode for Carrying Out the Invention

A method for improving physiological state of a subject, an apparatus for improving physiological state by light stimulation configured to irradiate light of a specific wavelength to a living body which produces an effect of improving physiological state, a method of operating the apparatus for improving physiological state by light stimulation configured to irradiate light of a specific wavelength to a living body which produces an effect of improving physiological state, and a computer program which causes to execute the method of operating will be described below while referring to the accompanying diagrams. However, the present invention is not restricted to content of embodiments and examples described below, and includes various modified examples and application examples within the scope of the present invention.

### [Method for improving physiological state of subject]

The method for improving physiological state of a subject according to the present disclosure is characterized by including irradiating light of a specific wavelength to the subject.

The inventors of the present invention discovered that by irradiating light of a specific wavelength to a subject, it is possible to improve physiological state of the subject. Here, improvement of physiological state may be at least one selected from a group consisting of improvement or suppression of dermatitis, improvement or suppression of alopecia, improvement of hair condition, improvement or suppression of graying of hair, improvement or suppression of hearing loss, improvement or suppression of vision loss, improvement or enhancement of muscle strength, improvement or suppression of kyphosis, improvement or suppression of pain and/or feeling of discomfort, improvement of autonomic nerve abnormalities, improvement of healthy life expectancy, and obesity control. Therefore, one aspect of the present disclosure is related to a method for improving physiological state of a subject, which includes irradiating light of a specific wavelength to the subject. In some embodiments, the improvement of physiological state may by at least one selected from a group consisting of improvement or suppression of dermatitis, improvement or suppression of alopecia, improvement of hair condition, improvement or suppression of graying of hair, improvement or suppression of hearing loss, improvement or suppression of vision loss, improvement or enhancement of muscle strength, improvement or suppression of kyphosis, improvement or suppression of pain and/or feeling of discomfort, improvement of autonomic nerve abnormalities, improvement of healthy life expectancy, and obesity control. Here, the subject may be an animal such as a mammal, particularly a human.

Some embodiments according to the present disclosure are related to a method for improving or suppressing dermatitis of a subject, which includes irradiating light of a specific wavelength to the subject. In some embodiments, dermatitis includes for example, atopic dermatitis, autoimmune dermatitis, contact dermatitis, viral dermatitis, seborrheic eczema, asteatotic eczema, hand eczema, diaper dermatitis, autosensitization dermatitis, nummular eczema, dyshidrotic eczema, sudamen, intertrigo, and solar dermatitis, but is not restricted thereto. The method according to the present disclosure may further include administering medication such as steroids, antiallergic drugs, immunosuppressive drugs, antibiotics, antimycotic drugs, antirespirants, moisturizing agents, and/or vitamin preparations.

Some embodiments of the present disclosure are related to a method for improving or suppressing alopecia of the subject which includes irradiating light of a specific wavelength to the subject. In some embodiments, alopecia includes for example, male pattern alopecia, female pattern alopecia, seborrheic alopecia, senile alopecia, alopecia areata, drug-induced alopecia, anticancer drug induced alopecia, X-ray induced alopecia, cicatrical alopecia, and postpartum alopecia after delivery, but is not restricted thereto. The method according to the present disclosure may further include administering vasodilating agents (for example, minoxidil), DHT generation inhibitor (for example, finasteride), steroids, carpronium chloride, glycyrrhizin, and/or cephalanthine.

Some embodiments according to the present disclosure are related to a method for improving coat condition of the subject, which includes irradiating light of a specific wavelength to the subject. In some embodiments, the improvement of hair condition includes for example, improvement of hair moisture, improvement of hair dryness, improvement of feel of hair, improvement of hair luster, and improvement of split ends of hair, but is not restricted thereto. The method according to the present disclosure may further include applying a hair treatment agent and a hair conditioner to the subject.

Some embodiments of the present disclosure are related to a method for improving or suppressing graying of hair of a subject, which includes irradiating light of a specific wavelength to the subject.

Some embodiments of the present disclosure are related to a method for improving or suppressing hearing loss of a subject, which includes irradiating light of a specific wavelength to the subject. In some embodiments, the hearing loss includes for example, age-associated hearing loss, sudden hearing loss, acute low-tone sensorineural hearing loss, noise-induced hearing loss, steroid-dependent hearing loss, acute sensorineural hearing loss, conductive hearing loss, sensorineural hearing loss, drug-induced sensorineural hearing loss, or mixed hearing loss, but is not restricted thereto. The method according to the present disclosure may further include administering for example, steroids, vitamin B12 preparations, ATP preparations, and/or osmotic diuretics to the subject.

Some embodiments of the present disclosure are related to a method for improving or suppressing vision loss of a subject, which includes irradiating light of a specific wavelength to the subject.

Some embodiments of the present disclosure are related to a method for improving or enhancing muscle strength of a subject, which includes irradiating light of a specific wavelength to the subject. In some embodiments, improving or enhancing muscle strength includes improving or enhancing muscle strength of patients suffering from sarcopenia, muscular dystrophy, spinocerebellar degeneration, amyotrophic lateral sclerosis, and/or congenital myopathy, but is not restricted thereto.

Some embodiment according to the present disclosure are related to a method for improving or suppressing kyphosis of a subject, which includes irradiating light of a specific wavelength to the subject. In some embodiments, the method for improving or suppressing kyphosis includes improving or suppressing kyphosis of patients suffering from osteoporosis, dysosteogenesis, osteoarthritis of the knee, and osteoarthritis of the spine, but is not restricted thereto. The method according to the present disclosure may further include administering calcium preparations, active vitamin D3 preparations, vitamin K2 preparations, female hormone preparations (estrogen), bisphosphonate preparations, SERM (Selective Estrogen Receptor Modulator) (raloxifene hydrochloride and bazedoxifene acetate), calcitone preparations, parathormones (for example, teriparatide), and/or anti-Lancourt antibody drug (for example, denosumab).

Some embodiments according to the present disclosure are related to a method for improving or suppressing pain and/or feeling of discomfort, which includes irradiating light of a specific wavelength to the subject. In some embodiments, the pain and/or feeling of discomfort may be due to nociceptive pain or neuropathic pain. The method according to the present disclosure may further include administering for example, non-steroidal anti-inflammatory drugs (NSAIDs), neuropathic pain treatment drugs, opioids, analgesic adjuvants, steroids, and/or anesthetics to the subj ect.

Some embodiments of the present disclosure are related to a method for improving autonomic nerve abnormalities of a subject, which includes irradiating light of a specific wavelength to the subject. In some embodiments, the autonomic nerve abnormalities may be due to autonomic nervous disorder. The method according to the present disclosure may further include administering for example, anti-anxiety drugs (tranquilizers), antidepressants, and/or sleep-inducing drugs to the subj ect.

Some embodiments of the present disclosure are related to a method for improving healthy life expectancy of a subject, which includes irradiating light of a specific wavelength to the subject.

Some embodiments of the present disclosure are related to a method for obesity control of a subject, which includes irradiating light of a specific wavelength to the subj ect.

In some embodiments of the method according to the present disclosure, the light used may be violet light. Moreover, in some embodiments of the method according to the present disclosure, the specific wavelength used may include a wavelength in a range of 350 nm to 400 nm, and may be approximately 380 nm specifically. The blinking frequency, for example, may be 0 Hz or a frequency in a range of 30 Hz to 75 Hz, and 0 Hz or a frequency in a range of 35 Hz to 60 Hz specifically. The blinking frequency may be 0 Hz or approximately 40 Hz specifically. The irradiation conditions may further include an irradiation time of the light source. The light source may be spectacles with a light source or a spectacle frame with a light source, a desktop light, a mobile terminal mounted light source, a light source installed nearby or in front of face, a portable light source, an indoor lighting, and a desk lamp, but is not restricted thereto.

In some embodiments of the present disclosure, the specific time of irradiating light may be an arbitrary time in a range of 10 seconds to 24 hours per day, and may be any one of 10 seconds, 30 seconds, 45 seconds, one minute, three minutes, five minutes, 10 minutes, 15 minutes, 30 minutes, 45 minutes, one hour, two hours, three hours, four hours, five hours, six hours, seven hours, eight hours, nine hours, 10 hours, 11 hours, 12 hours, 18 hours, and 24 hours, or may be an arbitrary time included in in a range (for example, a range of one hour to 12 hours) stipulated by the abovementioned arbitrary time. The specific period for which the light is irradiated continuously may be, for example, one day, two days, three days, four days, five days, six days, one week, two weeks, three weeks, one month, two months, three months, six months, one year, two years, three years, or a period longer than these periods.

One aspect of the present disclosure is related to a method for improving physiological state of a subject which includes improving physiological state of the subject by irradiating light of a specific wavelength to the subject by controlling a light irradiating apparatus. In some embodiments, the light irradiating apparatus is capable of irradiating violet light. Moreover, in some embodiment, the light irradiating apparatus is capable of irradiating light of a wavelength in a range of 350 nm to 400 nm, and specifically, light of a wavelength of approximately 380 nm. The blinking frequency of the light irradiating apparatus may be controllable to 0 Hz or in a range of 30 Hz to 75 Hz for example, and specifically, to 0 Hz or in a range of 35 Hz to 60 Hz, and the blinking frequency specifically, may be 0 Hz or approximately 40 Hz. The irradiation time of the light irradiating apparatus may be controllable. The light source may be in the form of spectacles with a light source or a spectacle frame with a light source, a desktop light source, a mobile terminal mounted light source, a light source installed nearby or in front of face, a portable light source, an indoor lighting, or a desk lamp, but is not restricted thereto.

### [Apparatus]

One aspect of the present disclosure is related to an apparatus for improving physiological state by light stimulation. In some embodiments, the apparatus for improving physiological state according to the present disclosure may include at least one light source which emits light, and a driving circuit which drives the light source. Here, the light emitted by the light source is a light of specific wavelength which produces an effect of improving physiological state by being irradiated to a living body. In some embodiments, the apparatus for improving physiological state is configured to irradiate light of a specific wavelength which produces the effect of improving physiological state of a subject.

In the present specification, the apparatus used for improving physiological state by light stimulation is referred to as `apparatus for improving physiological state' or just as 'apparatus'.

These apparatuses are capable of improving physiological state of a living body by controlling irradiation state of light.

In some embodiments, the apparatus according to the present disclosure, as mentioned above, is an apparatus for improving physiological state of a subject by irradiating violet light constantly or at a specific blinking frequency to a living body, and is characterized by including a light source which emits the violet light and a light-emission cycle controller which makes irradiate the violet light constantly or at a specific blinking frequency, and a light-emission time controller which makes irradiate the violet light for a specific time or for a specific period of time, and by being used for improving the physiological state.

Moreover, the apparatus according to the present disclosure, in some embodiments, is an apparatus for improving physiological state by irradiating violet light constantly to a living body, and is characterized by including a light source which emits the violet light, and a light-emission time controller which makes irradiate the violet light for a specific time or for a specific period of time.

Moreover, the apparatus according to the present disclosure, in some embodiments, is related to an apparatus used for improving physiological state by light stimulation which includes at least one light source which emits light, and a driving circuit which drives the light source, and the light emitted by the light source is light of a specific wavelength which produces the effect by being irradiated to a living body. Therefore, one aspect of the present disclosure is related to an apparatus for improving physiological state by light stimulation which is configured to include at least one light source which emits light and a driving circuit which drives the light source, and the driving circuit includes at least one processor which is communicably connected to the light source and at least one memory, and at least one memory for storing commands that are executable by the processor, and the light source emits light of a specific wavelength which produces an effect of improving physiological state by being irradiated to a living body.

### (Light source)

The wavelength of light emitted by the light source is not restricted, and in some embodiments, violet light defined by a wavelength range of 360 nm to 400 nm is used.

A light source with a possible oscillating frequency in a range of 0 Hz (constantly irradiated light, direct light) to 150 Hz can be used preferably. The frequency can be adjusted in units of 0.5 Hz and 1 Hz by setting the controller, and light of an arbitrary blinking frequency can be produces. As the blinking frequency is increased, there is an advantage that the blinking is not sensed any more, although this perception varies from person to person. The blinking frequency is not restricted to 10 Hz and 60 Hz used in experimental examples.

The irradiance from the light source may be variable or may be a constant value. Although irradiance with maximum output of 310 µW/cm² is used in some embodiments, it is not restricted to this value. The light source can be configured arbitrarily for irradiance in a range of 0.1 µW/cm² (0.001 W/m²) to 5000 µW/cm² (50 W/m²) for example, or irradiance in a range of 1 µW/cm² (0.01 W/m²) to 1000 µW/cm² (10 W/m²) for example, or irradiance in a range of 0.5 µW/cm² (0.005 W/m²) to 500 µW/cm² (5 W/m²) for example, or irradiance in a range of 0.5 µW/cm² to 1000 µW/cm². Furthermore, since a light source with such irradiance can be used easily for spectacles or a spectacle frame, and other portable irradiation equipment, it can be worn even in a day-to-day life. Even with weak light of extremely small amount (light with weak optical sensitivity) in particular, an occurrence of a characteristic phenomenon has been verified, and an effect on various parts of a living body including brain, and an application in cell activity (used with a significance including gene expression control) can be anticipated.

Light may be specified in terms of relative luminosity. Since characteristics of the present invention can be realized even with a low relative luminosity, the violet light which generates stimulation in a living body can be irradiated while blinking at a low relative luminosity, and a desired site can be stimulated without strain on a living body.

It is preferable to set the irradiation time of light arbitrarily according to the purpose thereof, and it may be a short time or a long time. The light can be irradiated intermittently (at regular interval or irregular interval) or continuously. The time of irradiation of light can be set to be a period of time between 8 am to 1 pm, between 9 am to 12 pm, or between 10 am to 11 am, and the period can be set to be at least 10 minutes, at least 15 minutes, at least 20 minutes, at least 30 minutes, at least one hour, at least two hours, at least three hours, at least four hours, or at least five hours. In some embodiments, irradiation for a period of three hours from 9 am to 12 pm, or for a period of two hours from 9 am to 11 am is used. In a case of irradiation in which the time has been set in this manner, a timer function can be used.

The light source may be spectacles or a spectacle frame with a light source. Such spectacles or spectacle frame being easy to wear, and having a light source which emits light at a blinking frequency fitted to spectacles or spectacle frame with no uncomfortable feeling on a daily basis, they are highly practical, and can be worn any time even in various situations and varied environments. Moreover, the light source may be a desktop light source, or a light source installed nearby or in front of face such as a mobile terminal mounted light source, or a non-mounting type light source such as a portable light source, or may be a mounting type light source such as an indoor lighting, a table stand, and a dedicated device, and can be an apparatus in various forms of light source appropriate for an environment of usage.

### (Controller)

The controller is a section which controls irradiation conditions (irradiate constantly or at a blinking frequency) of light from the light source. The controller may be a controller provided with an electric power source for supplying electric power to the light source, and such electric power source may be a battery, or may have a wire drawn to a battery installed at a different location. Moreover, in a case of being immovable at one location, it may be connected to a household electric power supply.

It is preferable that the controller, by tranceiving to and from an isolated controller such as a portable terminal, changes irradiation conditions of light such as blinking frequency, irradiance, irradiation time, irradiation-start time, and irradiation-end time. Since such controller carries out an isolated control of various irradiation conditions mentioned above, it is possible to have the desired effect by setting arbitrarily the irradiation conditions appropriate from controlling the desired biological function.

Furthermore, the controller may have controller functions and timer functions of the light source. The controller functions include functions such as changing the frequency and irradiance, and setting the irradiation time. Moreover, the time functions include an ability to set the irradiance time of light. Such controller functions and timer functions may have been provided integrally with the instrument, or may be provided as separate members.

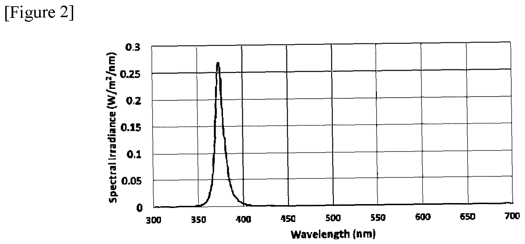

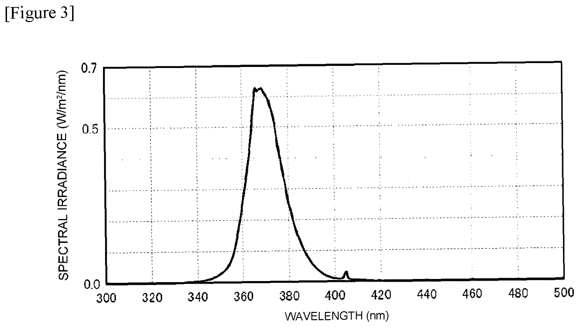

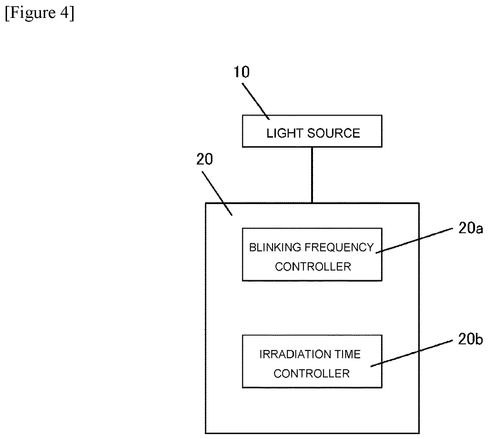

A block diagram of a simplified example of an apparatus which can be used for the abovementioned control of biological functions as an embodiment of the apparatus according to the present disclosure is shown in Fig. 4. The apparatus shown in Fig. 4 is an apparatus which may include various functions of the apparatus for improving physiological state of a subject by light stimulation described in the present specification, and therefore all the abovementioned apparatuses in the present specification can be represented by the block diagram in Fig. 4. The apparatus according to the present disclosure can have a light source 10 and a controller 20. The light source 10 irradiates light of a specific wavelength. It is preferable that a wavelength of the light irradiated by the light source 10 includes VL (violet light) or a wavelength range of 350 nm to 400 nm described heretofore, and more preferably, includes a wavelength of 380 nm. The light source 10 may be an arbitrary light source, and a light emitting diode (LED) can be used preferably from viewpoints such as small size, long life, and ease of blinking control (refer to Fig. 2 for an example of a spectrum of violet fluorescent light and Fig. 3 for an example of a spectrum of an LED). The number of light sources 10 may be one or may be plural depending on factors such as an intended intensity of irradiation and an intended range of irradiation of the light source.

The controller 20 is connected to the light source 10 by a wired connection or a wireless connection, and is configured to control irradiation conditions of the light source 10. The irradiation conditions can include at least one of the blinking frequency and the irradiation time of the light source 10, and therefore, the controller 20 can include at least one of a blinking frequency controller 20a and an irradiation time controller 20b. The blinking frequency may be preferably 0 Hz or a frequency in a range of 30 Hz to 75 Hz, and more preferably 0 Hz or a frequency in a range of 35 Hz to 45 Hz, and even more preferably 0 Hz or 40 Hz specifically. The blinking frequency 0 Hz refers to constantly irradiated light. The irradiation time can be set arbitrarily to be in a range of 10 seconds to 24 hours per day for example, and a specific period of time for which the irradiation is to be continued can be set arbitrarily for a period of one day to few years, or more than that for example.

The controller 20 can include a processor such as a CPU (Central Processing Unit), and executes a processing of controlling irradiation conditions of the light source 10. The processing carried out by the controller 20 may be realized by a computer program or may be realized by hardware by a logic circuit. The computer program may be stored in a computer-readable recording medium. The recording medium having the computer program stored may be a non-transient recording medium. The non-transient recording medium is not restricted in particular, and may be a recording medium such as a memory card and a CD-ROM (Compact Disc - Read Only Memory). The computer program stored in the recording medium can be installed in a computer unit via an appropriate reader. Examples of appropriate reader are a card reader in a case in which the recording medium is a memory card and a CD (Compact Disc) drive in a case in which the recording medium is a CD-ROM. Moreover, the computer program may be a computer program downloaded to a computer unit via a communication network from an external server.

In the apparatus according to the present disclosure, the driving circuit may include at least one processor which is communicably connected to the light source and at least one memory, and at least one memory for storing commands that are executable by the processor.

The light source 10 of the apparatus shown in Fig. 4 may be spectacles with a light source or a spectacle frame with a light source, a desktop light source, a mobile terminal mounted light source, a light source installed nearby or in front of face, a portable light source, an indoor lighting, or a table stand. Moreover, the apparatus according to the present disclosure may be provided as a product with a light source having at least the light source 10 mounted thereon, out of the light source 10 and the controller 20, including spectacles or a spectacle frame, a desktop light, a mobile terminal, a case for a mobile terminal, a head-worn article (such as cap, earphone headphone), a portable light, an indoor lighting, or a table stand.

As described heretofore, the apparatus according to the present disclosure can lead to the improvement of physiological state of a subject by irradiating light of a specific wavelength such as violet light constantly or at a specific blinking frequency to a living body.

Moreover, one aspect of the present invention is related to a method of operating an apparatus for improving physiological state by light stimulation. Accordingly, some embodiments of the present disclosure are related to a method of operating the apparatus used for improving physiological state of a subject, and the apparatus includes a light source which irradiates light of a specific wavelength constantly or at a specific blinking frequency to a living body, and a controller which controls the blinking frequency of the light source, and the apparatus irradiates light of a specific wavelength constantly or at a specific blinking frequency to a living body. Here, the blinking frequency of the light source may be controlled to be 0 Hz or to be in a range of 30 Hz to 75 Hz, and moreover, the light source may be a light source which irradiates light of a wavelength in a range of 350 nm to 400 nm to a living body. In addition, one aspect of the present invention is related to a computer program which causes an apparatus including a light source which irradiates light of a specific wavelength constantly or at a specific blinking frequency to a living body, and a controller which controls a blinking frequency of the light source, to execute the method of operating.

The computer program according to the present disclosure may have a command stored in a non-transitory computer-readable medium. The computer program according to the present disclosure can execute predetermined steps when the command is executed by the processor. Accordingly, one aspect of the present disclosure is related also to a computer-readable medium which is a non-transitory computer-readable medium having a command stored therein, and as the command is executed by a processor, it is capable of executing for an apparatus including a light source which irradiates light of a specific wavelength constantly or at a specific blinking frequency to a living body, and a controller which controls a blinking frequency of the light source, a step of operating the apparatus to control the blinking frequency of the light source to 0 Hz or to be in a range of 30 Hz to 75 Hz by the controller, and a step of operating the apparatus such that the light source irradiates light of a wavelength in a range of 350 nm to 400 nm to a living body.

Furthermore, one aspect of the present invention is related to an instrument for improving physiological state by light stimulation, and the instrument may include a glass, a spectacle lens, or a contact lens which allows violet light to pass through. By using such instrument, it is possible to have a preferable effect such as improvement or suppression of dermatitis, improvement or suppression of alopecia, improvement of hair condition, improvement or suppression of graying of hair, improvement or suppression of hearing loss, improvement or suppression of vision loss, improvement or enhancement of muscle strength, improvement or suppression of kyphosis, improvement and suppression of pain and/or feeling of discomfort, improvement of autonomic nerve abnormalities, improvement of healthy life expectancy, and obesity control. By using such instrument, improvement of physiological state of a subject may be possible.

### [Examples]

### (Light irradiation on mouse)

A mouse cage was placed in a light exposure device (New Opto, Kanagawa, Japan) having LEDs (white: NF2W757GT-F1, purple: NSSU123, Nichia Corporation, Tokushima) attached to an upper portion of a box. Mice of a control group were reared in white light (WL) from 8 am to 8pm in a cycle of 12-hour light period / 12-hour dark period. Mice of an experimental group as well, were exposed to white light under the same conditions, except for an exposure to violet light (VL). In other words, violet light (VL) was irradiated to the mice in the experimental group from 8 am till 8 pm. For violet light, light of a wavelength in a range of 360 nm to 400 nm (peak wavelength 375 nm) was used, and was irradiated constantly (blinking frequency of 0 Hz) in a range of irradiance of 0.5 µW/cm² to 1000 µW/cm². Irradiation of WL/VL was started at the age of 34 weeks, and frailty index test was carried out at the age of 92 weeks or 100 weeks.

### (Frailty index test at age of 92 weeks)

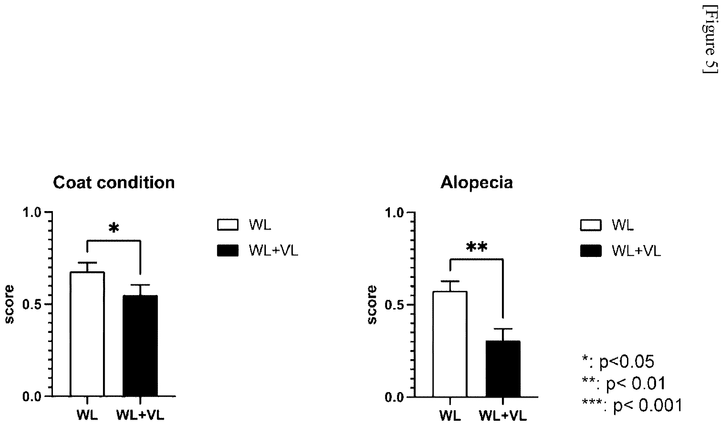

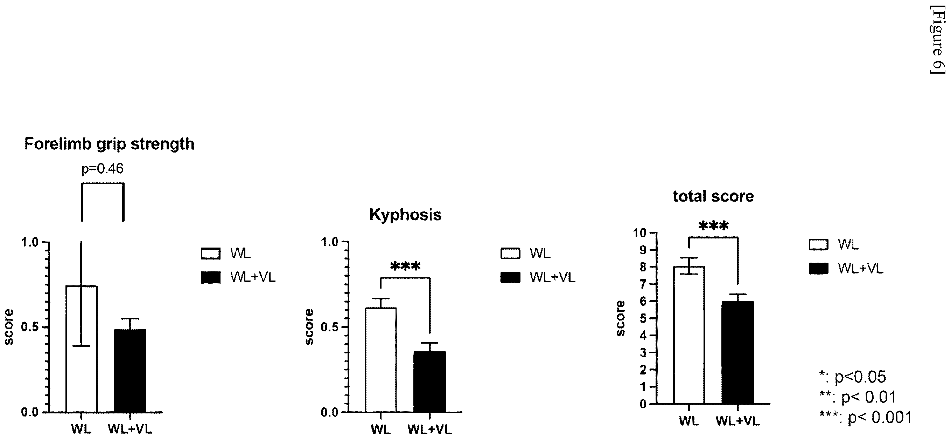

Results of frailty index test at the age of 92 weeks are shown in Fig. 5 and Fig. 6. A graph on a left side in Fig. 5 shows a comparison of coat condition of mice in the control group (WL) and the VL irradiation group (WL+VL). Values on a vertical axis are clinical condition observed in an mice subjected to numerical conversion, and are represented numerically where, 0 indicates `no symptoms', 0.5 indicates `mild symptoms', and 1 indicates `severe symptoms'. Accordingly, the higher the score more is the aging. The results in Fig. 5 indicate that due to irradiation of VL, aging of coat condition has been suppressed significantly. Accordingly, from these results, it is understood that by irradiation of VL, the improvement of hair condition is possible in human.

A graph on a right side in Fig. 5 shows a comparison of alopecia in mice in the control group (WL) and the VL irradiation group (WL+VL). This alopecia is also associated with dermatitis. The results in Fig. 5 indicate that due to irradiation of VL, alopecia and dermatitis associated therewith are suppressed significantly. Accordingly, from these results, it is understood that by irradiation of VL, the improvement or suppression of alopecia and dermatitis is possible even in human.

A graph on a left side in Fig. 6 shows a comparison of forelimb grip strength of mice in the control group (WL) and the VL irradiation group (WL+VL). The results in Fig. 6 indicate that due to irradiation of VL, weakening of the forelimb grip strength is suppressed significantly. Accordingly, from these results, it is understood that by irradiation of VL, the improvement or enhancement of muscle strength is possible even in human.

A graph at a center in Fig. 6 shows a comparison of kyphosis in mice in the control group (WL) and the VL irradiation group (WL+VL). The results in Fig. 6 indicate that due to irradiation of VL, kyphosis is suppressed significantly. Accordingly, from these results, it is understood that by irradiation of VL, the improvement or suppression of kyphosis is possible even in human.

A graph on a right side in Fig. 6 shows a comparison of a total score of frailty index test in the control group (WL) and the VL irradiation group (WL+VL). The results in Fig. 6 indicate that due to irradiation of VL, the total score which signifies the healthy life expectancy is suppressed significantly. Accordingly, from these results, it is understood that by irradiation of VL, the improvement of healthy life expectancy is possible even in human.

### (Frailty index test at age of 100 weeks)

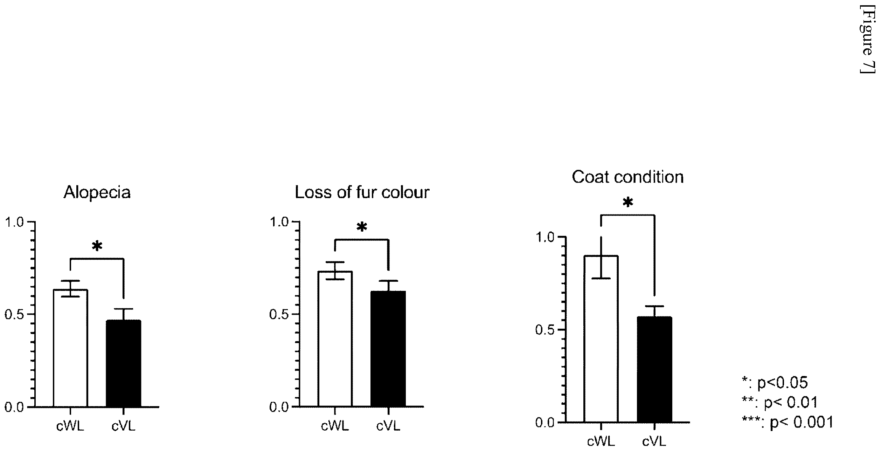

Results of frailty index test at the age of 100 weeks are shown in Fig. 7 to Fig. 10. A graph on a left side in Fig. 7 shows a comparison of alopecia in mice in the control group (WL) and the VL irradiation group (WL+VL). This alopecia is associated with dermatitis. The results in Fig. 7, similarly as the results for 92-week-old, indicate that due to irradiation of VL, alopecia and dermatitis associated therewith are suppressed significantly. Accordingly, from these results, it is understood that by irradiation of VL, the improvement or suppression of alopecia and dermatitis is possible even in human.

A graph at a center in Fig. 7 shows a comparison of a loss of fur color of mice in the control group (WL) and the VL irradiation group (WL+VL). The results in Fig. 7 indicate that due to irradiation of VL, the loss of fur color is suppressed significantly. Accordingly, from these results, it is understood that by irradiation of VL, the improvement or suppressing of graying of hair is possible even in human.

A graph on a right side in Fig. 7 shows a comparison of coat condition of mice in the control group (WL) and the VL irradiation group (WL+VL). The results in Fig. 7, similar to the results for 92-week-old, indicate that due to irradiation of VL, the aging of coat condition is suppressed significantly. Accordingly, from these results, it is understood that by irradiation of VL, the improvement of hair condition is possible in human.

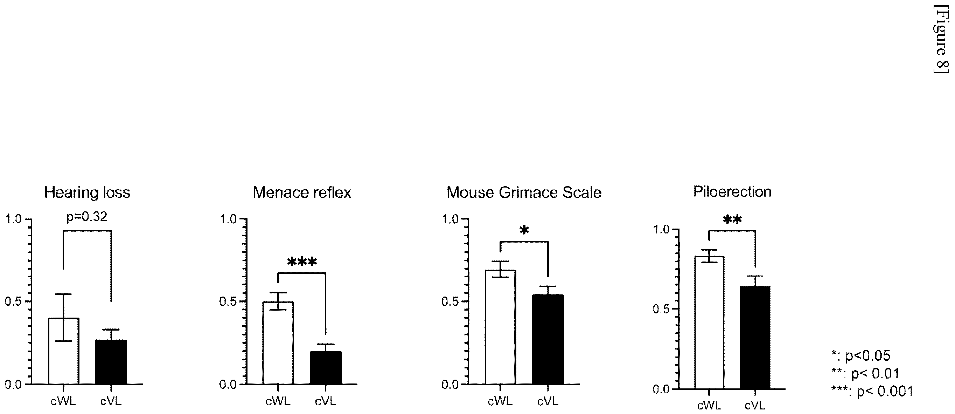

A graph on an extreme left in Fig. 8 shows a comparison of hearing loss in mice of in the control group (WL) and the VL irradiation group (WL+VL). The results in Fig. 8 indicate that due to irradiation of VL, the hearing loss is suppressed significantly. Accordingly, from these results, it is understood that by irradiation of VL, the improvement or suppression of hearing loss is possible even in human.

A second graph from a left side in Fig. 8 shows a comparison of menace reflex in mice in the control group (WL) and the VL irradiation group (WL+VL). The results in Fig. 8 indicate that due to VL irradiation, degradation of menace reflex (which corresponds to degradation of vision) is suppressed significantly. Accordingly, from these results, it is understood that by irradiation of VL, the improvement or suppression of vision loss is possible even in human.

A second graph from a right side in Fig. 8 shows a comparison of mouse grimace scale in mice in the control group (WL) and the VL irradiation group (WL+VL). The results in Fig. 8 indicate that due to VL irradiation, degradation of mouse grimace scale (which corresponds to reduction of pain and/or feeling of discomfort) is suppressed significantly. Accordingly, from these results, it is understood that by irradiation of VL, that the improvement or suppression of pain and/or feeling of discomfort is possible even in human.

A graph on an extreme right side in Fig. 8 shows a comparison of piloerection in mice in the control group (WL) and the VL irradiation group (WL+VL). The results in Fig. 8 indicate that due to irradiation of VL, degradation of piloerection (which corresponds to improvement of autonomic nerve abnormalities and reduction of feeling of discomfort) is suppressed significantly. Accordingly, from these results, it is understood that by irradiation of VL, the improvement of autonomic nerve abnormalities and improvement or suppression of feeling of discomfort are possible even in human.

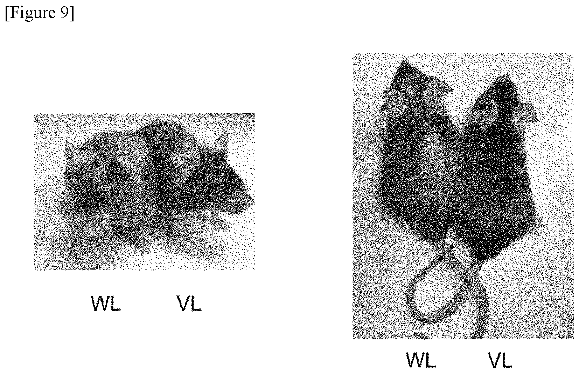

Photographs in Fig. 9 show a comparison of alopecia and gray hair in mice in the control group (WL) and the VL irradiation group (WL+VL). The results in Fig. 9 indicate that due to irradiation of VL, alopecia and gray hair have reduced. Accordingly, from these results, it is understood that by irradiation of VL, the improvement of hair condition is possible in human.

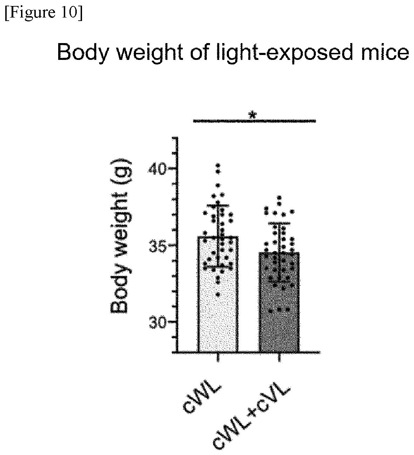

A graph in Fig. 10 shows a comparison of body weight of mice in the control group (cWL) and the VL irradiation group (cWL+cVL). The results in Fig. 10 indicate that due to irradiation of VL, obesity is controlled significantly. Accordingly, from these results, it is understood that by irradiation of VL, obesity control is possible even in human.

All publications, applications, standards, and patents mentioned in the present specification are herein incorporated by reference in its entirety, and in a case of contradictory terminology, the present specification will take precedence. Scope of the present invention is not to be restricted by specific embodiments described in the present specification. In fact, in addition to the description in the present specification, various modifications of the present invention will be apparent to the person skilled in the art from the abovementioned description and the accompanying drawings. Such amendments are intended to fall within the scope of the appended patent claims. Some or all of the embodiments described heretofore may also be described as the following supplementary notes, and the disclosure of the present application is not restricted to the following supplementary notes.

(Supplementary Note 1)
   A method for improving physiological state of a subject, comprising:
   irradiating light of a specific wavelength to the subject.
(Supplementary Note 2)
   A method for improving physiological state of a subject, comprising:
   irradiating light of a specific wavelength to the subject by controlling a light irradiating apparatus, thereby improving physiological state of a subject.
(Supplementary Note 3)
   The method according to supplementary note 2, wherein the light irradiating apparatus comprises a light source which irradiates light of a specific wavelength constantly or at a specific blinking frequency to a living body, and a controller which controls the blinking frequency of the light source.
(Supplementary Note 4)
   The method according to any one of supplementary notes 1 to 3, wherein the improvement of physiological state is at least one selected from a group consisting of
   improvement or suppression of dermatitis,
   improvement or suppression of alopecia,
   improvement of hair condition,
   improvement or suppression of graying of hair,
   improvement or suppression of hearing loss,
   improvement or suppression of vision loss,
   improvement or enhancement of muscular strength,
   improvement or suppression of kyphosis,
   improvement or suppression of pain and/or feeling of discomfort,
   improvement of autonomic nerve abnormalities,
   improvement of healthy life expectancy, and
   obesity control.
(Supplementary Note 5)
   The method according to any one of supplementary notes 1 to 4, wherein the light is violet light.
(Supplementary Note 6)
   The method according to any one of supplementary notes 1 to 5, wherein the specific wavelength includes a wavelength in a range of 350 nm to 400 nm.
(Supplementary Note 7)
   The method according to supplementary note 6, wherein the specific wavelength includes a wavelength of approximately 380 nm.
(Supplementary Note 8)
   The method according to any one of supplementary notes 1 to 7, wherein the blinking frequency is either 0 Hz or a frequency in a range of 30 Hz to 75 Hz.
(Supplementary Note 9)
   The method according to supplementary note 8, wherein the blinking frequency is either 0 Hz or a frequency in a range of 35 Hz to 60 Hz.
(Supplementary Note 10)
   The method according to supplementary note 9, wherein the blinking frequency is either 0 Hz or approximately 40 Hz.
(Supplementary Note 11)
   The method according to any one of supplementary notes 1 to 10, wherein irradiation conditions further comprise an irradiation time of the light source.
(Supplementary Note 12)
   The method according to any one of supplementary notes 1 to 11, wherein the light source is spectacles with a light source or a spectacle frame with a light source, a desktop light source, a mobile terminal mounted light source, a light source installed nearby or in front of a face, a portable light source, an indoor lighting, or a desk lamp.
(Supplementary Note 13)
   The method according to any one of claims 1 to 12, wherein the subject is a human.
(Supplementary Note 14)
   An apparatus for improving physiological state by light stimulation, comprising:
   at least one light source which emits light; and
   a driving circuit which drives the light source,
   wherein the light emitted by the light source is light of a specific wavelength which produces an effect of improving physiological state by being irradiated to a living body.
(Supplementary Note 15)
   The apparatus according to supplementary note 14, wherein the driving circuit comprises at least one processor which is communicably connected to the light source and at least one memory, and at least one memory for storing commands that are executable by the processor.
(Supplementary Note 16)
   The apparatus according to one of supplementary notes 14 and 15, wherein the improvement of physiological state is at least one selected from a group consisting of
   improvement or suppression of dermatitis,
   improvement or suppression of alopecia,
   improvement of hair condition,
   improvement or suppression of graying of hair,
   improvement or suppression of hearing loss,
   improvement or suppression of vision loss,
   improvement or enhancement of muscle strength,
   improvement or suppression of kyphosis,
   improvement or suppression of pain and/or feeling of discomfort, improvement of autonomic nerve abnormalities,
   improvement of healthy life expectancy, and
   obesity control.
(Supplementary Note 17)
   A method of operating an apparatus according to one of supplementary notes 14 to 16,
   wherein the apparatus comprises a light source which irradiates light of a specific wavelength constantly or at a specific blinking frequency to a living body and a controller which controls a blinking frequency of the light source, and
   wherein the method comprises:
      controlling the blinking frequency of the light source either to 0 Hz or to a frequency in a range of 30 Hz to 75 Hz by the controller; and
      irradiating light of a wavelength in a range of 350 nm to 400 nm by the light source to the living body.
(Supplementary Note 18)
   A computer program which causes an apparatus including a light source which irradiates light of a specific wavelength constantly or at a specific blinking frequency to a living body and a controller which controls the blinking frequency of the light source, to execute the method of operating according to supplementary note 17.
(Supplementary Note 19)
   An apparatus for improving physiological state by light stimulation, comprising:
   at least one light source which emits light; and
   a driving circuit which drives the light source,
   wherein the driving circuit comprises at least one processor which is communicably connected to the light source and at least one memory, and at least one memory for storing commands that are executable by the processor, and
   the light source is configured to emit light of a specific wavelength which produces an effect of improving physiological state by being irradiated to a living body.
(Supplementary Note 20)
   A computer-readable medium which is a non-transitory computer-readable medium having commands stored therein, and as the command is executed by a processor, it is capable of executing for an apparatus including a light source which irradiates light of a specific wavelength constantly or at a specific blinking frequency to a living body, and a controller which controls the blinking frequency of the light source:
   a step of operating the apparatus to control the blinking frequency of the light source to 0 Hz or to be in a range of 30 Hz to 75 Hz by the controller; and
   a step of operating the apparatus such that the light source irradiates light of a wavelength in a range of 350 nm to 400 nm to a living body.
(Supplementary Note 21)
   An instrument for improving physiological state by light stimulation, comprising:
   a glass, a spectacle lens, or a contact lens which allows violet light to pass through.
(Supplementary Note 22)
   A method for improving or suppressing atopic dermatitis, autoimmune dermatitis, contact dermatitis, viral dermatitis, seborrheic eczema, asteatotic eczema, hand eczema, diaper dermatitis, autosensitization dermatitis, nummular eczema, dyshidrotic eczema, sudamen, intertrigo, and/or solar dermatitis of a subject, comprising:
   irradiating light of a specific wavelength to the subject.
(Supplementary Note 23)
   A method for improving or suppressing male pattern alopecia, female pattern alopecia, seborrheic alopecia, senile alopecia, alopecia areata, drug-induced alopecia, anticancer drug induced alopecia, X-ray induced alopecia, cicatrical alopecia, and/or postpartum alopecia after delivery of a subject, comprising:
   irradiation of light of a specific wavelength to the subject.
(Supplementary Note 24)
   A method for improvement of hair moisture, improvement of hair dryness, improvement of feel of hair, improvement of hair luster, and/or improvement of split ends of hair of a subject, comprising:
   irradiating light of a specific wavelength to the subject.
(Supplementary Note 25)
   A method for improving or suppressing age-associated hearing loss, sudden hearing loss, acute low-tone sensorineural hearing loss, noise-induced hearing loss, steroid-dependent hearing loss, acute sensorineural hearing loss, conductive hearing loss, sensorineural hearing loss, drug-induced hearing loss, or mixed hearing loss of a subject, comprising:
   irradiating light of a specific wavelength to the subject
(Supplementary Note 26)
   A method for improving or suppressing sarcopenia, muscular dystrophy, spinocerebellar degeneration, amyotrophic lateral sclerosis, and/or congenital myopathy of a subject, comprising:
   irradiating light of a specific wavelength to the subject.
(Supplementary Note 27)
   A method for improving or suppressing osteoporosis, dysosteogenesis, osteoarthritis of the knee, and/or osteoarthritis of the spine of a subject, comprising:
   irradiating light of a specific wavelength to the subject.
(Supplementary Note 28)
   A method for improving or suppressing nociceptive pain and/or neuropathic pain of a subject, comprising:
   irradiating light of a specific wavelength to the subject.
(Supplementary Note 29)
   A method for improving or suppressing autonomic nervous disorder of a subject, comprising:
   irradiating light of a specific wavelength to the subject.

## Claims

1. A method for improving physiological state of a subject, comprising:
irradiating light of a specific wavelength to the subject.

2. A method for improving physiological state of a subject, comprising:
irradiating light of a specific wavelength to the subject by controlling a light irradiating apparatus, thereby improving physiological state of a subject.

3. The method according to claim 2, wherein the light irradiating apparatus comprises a light source which irradiates light of a specific wavelength constantly or at a specific blinking frequency to a living body, and a controller which controls the blinking frequency of the light source.

4. The method according to any one of claims 1 to 3, wherein the improvement of physiological state is at least one selected from a group consisting of
improvement or suppression of dermatitis,
improvement or suppression of alopecia,
improvement of hair condition,
improvement or suppression of graying of hair,
improvement or suppression of hearing loss,
improvement or suppression of vision loss,
improvement or enhancement of muscle strength,
improvement or suppression of kyphosis,
improvement or suppression of pain and/or feeling of discomfort, improvement of autonomic nerve abnormalities,
improvement of healthy life expectancy, and
obesity control.

5. The method according to any one of claims 1 to 4, wherein the light is violet light.

6. The method according to any one of claims 1 to 5, wherein the specific wavelength includes a wavelength in a range of 350 nm to 400 nm.

7. The method according to claim 6, wherein the specific wavelength includes a wavelength of approximately 380 nm.

8. The method according to any one of claims 1 to 7, wherein the blinking frequency is either 0 Hz or a frequency in a range of 30 Hz to 75 Hz.

9. The method according to claim 8, wherein the blinking frequency is either 0 Hz or a frequency in a range of 35 Hz to 60 Hz.

10. The method according to claim 9, wherein the blinking frequency is either 0 Hz or approximately 40 Hz.

11. The method according to any one of claims 1 to 10 wherein irradiation conditions further comprise an irradiation time of the light source.

12. The method according to any one of claims 1 to 11, wherein the light source is spectacles with a light source or a spectacle frame with a light source, a desktop light source, a mobile terminal mounted light source, a light source installed nearby or in front of face, a portable light source, an indoor lighting, or a desk lamp.

13. The method according to any one of claims 1 to 12, wherein the subject is a human.

14. An apparatus for improving physiological state by light stimulation, comprising:
at least one light source which emits light; and
a driving circuit which drives the light source,
wherein the light emitted by the light source is light of a specific wavelength which produces an effect of improving a physiological state by being irradiated to a living body.

15. The apparatus according to claim 14, wherein the driving circuit comprises:
at least one processor which is communicably connected to the light source and at least one memory, and
at least one memory for storing commands that are executable by the processor.

16. The apparatus according to one of claims 14 and 15, wherein the improvement of physiological state is at least one selected from a group consisting of
improvement or suppression of dermatitis,
improvement or suppression of alopecia,
improvement of hair condition,
improvement or suppression of graying of hair,
improvement or suppression of hearing loss,
improvement or suppression of vision loss,
improvement or enhancement of muscle strength,
improvement or suppression of kyphosis,
improvement or suppression of pain and/or feeling of discomfort,
improvement of autonomic nerve abnormalities,
improvement of healthy life expectancy, and
obesity control.

17. A method of operating an apparatus according to any one of claims 14 to 16,
wherein the apparatus comprises a light source which irradiates light of a specific wavelength constantly or at a specific blinking frequency to a living body and a controller which controls a blinking frequency of the light source, and
wherein the method comprises:
controlling the blinking frequency of the light source either to 0 Hz or to a frequency in a range of 30 Hz to 75 Hz by the controller; and
irradiating light of a wavelength in a range of 350 nm to 400 nm by the light source to the living body.

18. A computer program which causes an apparatus including a light source which irradiates light of a specific wavelength constantly or at a specific blinking frequency to a living body and a controller which controls the blinking frequency of the light source, to execute the method of operating according to claim 17.
